# EUROPEAN PATENT APPLICATION

(11) **EP 0 552 545 A1**
(43) Date of publication of application: **28.07.1993**
(21) Application number: 92311242.9
(22) Date of filing: 09.12.1992
(51) Int. Cl.: C12Q 1/68

(54) **Detection of polymorphisms in simple sequence repeats using oligonucleotide ligation**

(30) Priority: 17.01.1992 US 826930
(71) Applicant: PIONEER HI-BRED INTERNATIONAL, INC., Des Moines Iowa 50309 (US)
(72) Inventor: Grant, David, Polk County, Iowa 50322 (US)
(74) Representative: Goldin, Douglas Michael

(57) **Abstract**

A method for detecting genetic differences using differences in simple sequence repeats as identified using ligation reaction-mediated detection.

## Description

### BACKGROUND OF THE INVENTION

### Technical Field

The present invention relates to the identification of genetic variations between different biological material samples.

### Background Art

Measurement of genetic identity and measurement of genetic diversity are two sides of the same coin. Each meaurement relies upon a technique which is capable of detecting differences and similarities between oligonucleotide sequences. The oligonucleotide sequences which are actually subjected to testing represent only a small fraction of the total genetic material, but are taken as a representative sample of the complete genome of the organism. Because of that sampling factor, it is important to assure that the sample is sufficiently large and sufficiently representative of the whole genome that the results obtained from the sample are an accurate representation of the results that would be obtained from the genome as a whole. The representational accuracy then is a function of numbers of oligonucleotide samples and the mapping of the oligonucleotide samples across the genome. Mapping, in turn, also requires large numbers of oligonucleotides be examined to establish relative positions and to assure a spread of samples across the genome.

Because numbers of oligonucleotide samples are important, the time and ease involved in procedures for the identification of oligonucleotides and matches or non-matches between oligonucleotides are also important. Current methods for measuring genetic identity or diversity are based on restriction fragment length polymorphisms as detected by Southern blot testing using gels. This method and all gel-based methods are expensive, laborious and time consuming.

It is important to develop a method of measuring genetic identity or diversity which is faster, cheaper and more amenable to automation than existing RFLP technologies. Several approaches have been suggested, many of which use some form of polymerase chain reaction amplification of oligonucleotides, including allele-specific PCR amplification and nested PCR amplification. Ligation of allele-specific oligonucleotides has also been suggested. While any of these methods offers some improvment over current gel-based RFLP methods, all of them nonetheless require that the sequence of every allele at any genetic locus of interest be known. In some species, such as maize (Zea mays L.) this represents a major disadvantage because there are on average 6 to 8 alleles at each of the approximately 1000 genetic loci in use, further amplifying the numbers of samples which must be run. Accordingly, a continuing need exists for an approach which would provide the ability to discriminate among numerous alleles at many genetic loci but does not require a priori knowledge of the DNA sequence for each allele. It is an object of this invention to provide such a method.

Simple sequence repeats (SSRs) are typically 1:1 tandem purine:pyrimidine dinucleotide repeats, usually GT or AC although CT and AT have been reported, as have 4:4 and 3:1 repeats. SSRs are ubiquitous in eukaryotes, although they are markedly underrepresented in bacteria (Sarkar et al., NAR 19:631 (1991)). In adition to being present in many thousands of copies per genome, SSRs are often highly polymorphic, the variation being in the number of repeats.

SSRs have been considered for use as genetic markers in man and mice. However, reported methods for detection of SSR variations are based either on direct sequencing of the repeat region or measurement of the numbr of repeats as variations in mobility on a sequencing gel of PCR amplified fragments. Both approaches are time consuming and not well-suited to large scale applications. Thus, a suitable procedure for exploiting SSRs in large-scale genetic testing has not yet been found.

### DISCLOSURE OF THE INVENTION

### Summary

The method of this invention takes advantage of the fact that, although variation in the number of repeats in the SSR is common, changes in the sequence of the DNA which flanks the SSR are not common. This can be illustrated by considering SSRs from two organisms:

### Organism A

...conserved.sequence.CACACACACACACACACA.CONSERVED.SEQUENCE...

### Organism B

...conserved.sequence.CACACACACACACA.CONSERVED.SEQUENCE...

### Step 1 - Amplification of fragments containing the SSRs

When PCR amplification is performed using oligonucleotides from the two flanking regions (conserved.sequence and CONSERVED.SEQUENCE) as primers, the amplification is not allele-specific. The PCR amplification will yield quantities of the two fragments:

### Organism A

conserved.sequence.CACACACACACACACACA.CONSERVED.SEQUENCE

### Organism B

conserved.sequence.CACACACACACACA.CONSERVED.SEQUENCE

### Step 2 - First ligation reaction

The two SSR alleles can then be detected and distinguished using a series of ligation reactions. The first ligation reaction uses two oligonuclotide fragments which are exactly complementary to the SSR in Organism A:
pair 1: sequence. and GTGTGTGTGTGTGTGTGT.CONSERVED Hybridization of this pair of oligonucleotides to the amplified genomic fragments obtained from Step 1 results in the following combinations:

### Organism A

sequence.GTGTGTGTGTGTGTGT.CONSERVED
conserved.sequence.CACACACACACACACA.CONSERVED.SEQUENCE

### Organism B

GTG
T
sequence.GTGTGTGTGTGTGT.CONSERVED
conserved.sequence.CACACACACACACA.CONSERVED.SEQUENCE

### Step 3 - Second ligation reaction

The second ligation reaction uses two oligonuclotice fragments which are exactly complementary to the SSR in Organism B:
pair 2: sequence. and GTGTGTGTGTGTGTGT.CONSERVED Hybridization of this pair of oligonucleotides to the amplified genomic fragments obtained from Step 1 results in the following combinations:

### Organism A

sequence.    GTGTGTGTGTGTGT.CONSERVED
conserved.sequence.CACACACACACACACACA.CONSERVED.SEQUENCE

### Organism B

sequence.GTGTGTGTGTGTGT.CONSERVED
conserved.sequence.CACACACACACACA.CONSERVED.SEQUENCE

It can be seen from this that the first oligonucleotide pair will successfully produce a ligation product with the fragment from Organism A, but not with the fragment from Organism B, while the opposite is true for oligonucleotide pair 2. This methodology can be applied to any simple sequence repeat which is flanked by sequences which are conserved from organism to organism. The sequences shown here are arbitrarily selected and are solely for purposes of illustration.

### Step 4 - Detection of the ligation reaction results

The ligation reactions can be read by techniques which are not gel-based, and thus can be automated easily. For example, one oligonucleotide in a pair can be operatively linked to a capture reagent, such as biotin, while the other oligonucleotide in the pair is operatively linked to a detection reagent, such as a chromophore or a fluorophore, or a chromogenic or fluorogenic moiety. Then detection can be performed simply by measurement of how much detection reagent is co-captured with the capture reagent. By "operatively linked" is meant that the reagent moiety is linked to the end of the oligonucleotide which does not interfere with the hybridization/ligation reaction, i.e., it is linked to the upstream end of the upstream conserved sequence portion or to the downstream end of the downstream conserved sequence portion. In general, the 5' end of a nucleotide sequence is considered the upstream end and the 3' end is considered the downstream end. This and other reaction product detection techiques are thoroughly familiar to those in the field of diagnostic and other bioreagent-based assays and by themselves are not a part of this invention.

### Advantages

Gel based detection systems are laborious and difficult to automate. This invention makes SSRs useful in the rapid, convenient detection of genetic polymormphisms because it permits detection of those polymorphisms without the use of gels.

In addition, only a single reference allele need be sequenced in order to identify the conserved flanking sequences. Since the alleles are defined in terms of variation in the number of repeats in the SSR, it is sufficient to accurately size the PCR product from each new allele and compare it to the reference allele product to determine this number. Appropriate complementary oligonucleotides can then be synthesized for each allele.

Although a new set of SSR based markers would be needed to create a genetic map, the Mouse Genome Project has found that the spacing and frequency of SSRs is sufficient to construct an arbitrarily dense genetic map using them. The convenience of using the method of this invention will permit such a map to be made more quickly and easily than with prior art methods.

## Claims

1. A method for detecting the presence or absence of genetic variation between a first organism and a second organism by detection of polymorphisms in a simple sequence repeat in the DNA of the organisms, comprising the steps of
a) identifying upstream and downstream flanking sequences with respect to the simple sequence repeat in the first organism which are conserved in the second organism;
b) for each of the first and second organisms, amplifying the simple sequence repeat from the genome of the organism using oligonucleotides from the conserved upstream and downstream flanking sequences as amplification primers to produce, for each organism, oligonucleotide fragments consisting of the simple sequence repeat from the organism and portions of the conserved upstream and downstream sequences corresponding to the amplification primers;
c) for the amplfied oligonucleotide fragments from each organism, reacting the oligonucleotide fragments with first and second pairs of detector molecules, the first pair of detector molecules comprising
i) a first nucleotide sequence complementary to either the upstream or downstream conserved sequence portion, and
ii) a second nucleotide sequence complementary to the other conserved sequence portion, linked in proper upstream-to- downstream sequence with the simple sequence repeat of the first organism;
whereby the first detector molecule pair hybridizes with the amplified oligonucleotide fragments from both organisms if there is no difference in the simple sequence repeats and successfully hybridizes only with the amplified oligonucleotide fragments from the first organism if there is a difference in the simple sequence repeats; and the second pair of detector molecules comprising
i) a third nucleotide sequence complementary to one of the upstream or downstream conserved sequence portion, and
ii) a fourth nucleotide sequence complementary to the other conserved sequence portion, linked in proper upstream-to-downstream sequence with the simple sequence repeat of the second organism
whereby the second detector molecule pair hybridizes with the amplified oligonucleotide fragments from both organisms if there is no difference in the simple sequence repeats and successfully hybridizes only with the amplified oligonucleotide fragments from the second organism if there is a difference in the simple sequence repeats; and
d) for the amplfied oligonucleotide fragments from each organism, detecting any differences between reaction products of the ligation reaction with the first pair of detector oligonucleotides and reaction products of the ligation reaction with the second pair of detector oligonucleotides; and
e) identifying differences in the results of the ligation reactions for the two organisms.

2. A method according to claim 1 wherein the one of the first and second detector molecules and one of the third and fourth detector molecules further comprises a capture reagent moiety operatively linked to the conserved sequence portion, and the other of the first and second detector molecules and the other of the third and fourth detector molecules comprises a detection reagent moiety operatively linked to the conserved sequence portion.

3. A method according to Claim 2 wherein the capture reagent moiety is biotin.

4. A method according to Claim 2 wherein the detection reagent moiety is a chromophore.


INFORMATION FOR SEQ ID NO:1:
(i) SEQUENCE CHARACTERISTICS:
(A) LENGTH: 18 base pairs
(B) TYPE: nucelotide
(C) STRANDEDNESS: single
(D) TOPOLOGY: linear
(ii) MOLECULE TYPE: SYNTHETIC DNA
(iii) HYPOTHETICAL: YES
(iv) ANTI-SENSE: NO
(xi) SEQUENCE DESCRIPTION: SEQ ID NO: 1:

CACACACACA CACACACA 18

INFORMATION FOR SEQ ID NO:2:
(i) SEQUENCE CHARACTERISTICS:
(A) LENGTH: 18 base pairs
(B) TYPE: nucelotide
(C) STRANDEDNESS: single
(D) TOPOLOGY: linear
(ii) MOLECULE TYPE: SYNTHETIC DNA
(iii) HYPOTHETICAL: YES
(iv) ANTI-SENSE: NO
(xi) SEQUENCE DESCRIPTION: SEQ ID NO: 2:

GTGTGTGTGT GTGTGTGT 18

INFORMATION FOR SEQ ID NO:3:
(i) SEQUENCE CHARACTERISTICS:
(A) LENGTH: 14 base pairs
(B) TYPE: nucelotide
(C) STRANDEDNESS: single
(D) TOPOLOGY: linear
(ii) MOLECULE TYPE: SYNTHETIC DNA
(iii) HYPOTHETICAL: YES

(iv) ANTI-SENSE: NO
(xi) SEQUENCE DESCRIPTION: SEQ ID NO: 3:

CACACACACA CACA 14

INFORMATION FOR SEQ ID NO:4:
(i) SEQUENCE CHARACTERISTICS:
(A) LENGTH: 14 base pairs
(B) TYPE: nucelotide
(C) STRANDEDNESS: single
(D) TOPOLOGY: linear
(ii) MOLECULE TYPE: SYNTHETIC DNA
(iii) HYPOTHETICAL: YES
(iv) ANTI-SENSE: NO
(xi) SEQUENCE DESCRIPTION: SEQ ID NO: 4:

GTGTGTGTGT GTGT 14